# EUROPÄISCHE PATENTSCHRIFT

(11) **EP 1 608 386 B1**
(45) Veröffentlichungstag und Bekanntmachung des Hinweises auf die Patenterteilung: **14.01.2009**
(21) Anmeldenummer: 04724553.5
(22) Anmeldetag: 31.03.2004
(51) Int. Cl.: A61K 36/55, A61K 36/535, A61K 35/60

(54) **VERWENDUNG OMEGA-3-FETTSÄUREHALTIGER ÖLE ZUR ERHÖHUNG DER BIOVERFÜGBARKEIT VON PFLANZENEXTRAKTEN**
USE OF OMEGA-3 FATTY ACID-CONTAINING OILS TO IMPROVE BIOAVAILABILITY OF PLANT EXTRACTS
UTILISATION DES HUILES CONTENANT DES ACIDES GRAS OMEGA-3 ET POUR AMELIORER LA BIODISPONIBILITE DES EXTRAITS VEGETAUX

(30) Priorität: 02.04.2003 DE 10315026
(43) Veröffentlichungstag der Anmeldung: 28.12.2005
(73) Patentinhaber: Dr. Willmar Schwabe GmbH & Co. KG, 76227 Karlsruhe (DE)
(72) Erfinder: BIBER, Anton, 76275 Ettlingen (DE); STUMPF, Karl-Heinz, 76228 Karlsruhe (DE)
(74) Vertreter: Adam, Holger
(86) Internationale Anmeldenummer: PCT/EP2004/003399
(87) Internationale Veröffentlichungsnummer: WO 2004/087180

(56) Entgegenhaltungen:
- WO-A-02/30404
- US-A1- 2002 004 074
- YOSHINO K ET AL: "ANTIOXIDATIVE AND HYPOLIPIDEMIC EFFECTS OF DIETARY GREEN TEA POLYPHENOLS IN DDY MICE FED FISH OIL-RICH DIET" SHOKUHIN EISEIGAKU ZASSHI, NIPPON SHOKUHIN EISEI GAKKAI, TOKYO, JP, Bd. 39, Nr. 3, Juni 1998 (1998-06), Seiten 192-198, XP009033072 ISSN: 0015-6426
- DATABASE WPI Section Ch, Week 200422 Derwent Publications Ltd., London, GB; Class B04, AN 2004-235875 XP002290854 & KR 2003 089 537 A (STK PHARM CO LTD) 22. November 2003 (2003-11-22)
- DATABASE WPI Section Ch, Week 200205 Derwent Publications Ltd., London, GB; Class B04, AN 2002-035004 XP002290855 & CN 1 311 028 A (WANG X) 5. September 2001 (2001-09-05)
- CARLSON JOSEPH J ET AL: "The effect of a supplement containing Ginkgo biloba (Gb), fish oil (DHA) and gotu kola (Gk) on platelet function in older healthy adults" FASEB JOURNAL, Bd. 16, Nr. 4, 20. März 2002 (2002-03-20), Seite A646, XP009034771 & ANNUAL MEETING OF THE PROFESSIONAL RESEARCH SCIENTISTS ON EXPERIMENTAL BIOLOGY; NEW ORLEANS, LOUISIANA, USA; APRIL 20-24, 2002 ISSN: 0892-6638

## Beschreibung

Die vorliegende Erfindung betrifft die Verwendung von omega-3-fettsäurehaltigen Ölen zur Erhöhung der Bioverfügbarkeit von sekundären Pflanzeninhaltsstoffen aus Pflanzenextrakten.

Der Begriff sekundäre Pflanzeninhaltsstoffe umfaßt diejenigen Inhaltsstoffe der Pflanze, die keine Bedeutung als Energieträger oder Gerüstsubstanzen haben. Der Pflanze dienen sie z. B. als Farbstoffe, Abwehrstoffe oder Lockstoffe. Die Zahl der sekundären Pflanzeninhaltsstoffe wird auf 10.000 bis 30.000 Einzelsubstanzen geschätzt. Aufgrund ihrer chemischen Struktur bzw. Biogenese kann man sie in folgende Gruppen fassen.

Polyphenole. Diese Gruppe umfaßt einfache Phenolcarbonsäuren wie z. B. Gentisinsäure, Protocatechusäure, Gallussäure oder Kaffeesäure, weiterhin Flavone wie z. B. Kämpferol, Quercetin, Myricetin, Isorhamnetin, Naringenin, 6-Prenylnaringenin, 8-Prenylnaringenin, Isoxanthohumol und deren Glykoside, Chalkone wie z. B. Xanthohumol, Isoflavone wie z. B. Daidzein und Genistein, Anthocyane wie z. B. Pelargonidin, Cyanidin, Malvidin oder Delphinidin, Gerbstoffe wie z. B. Catechin und Epicatechin sowie deren Oligomere und Polymere.

Isoprenoide. Diese Gruppe umfaßt alle sich von Isopren ableitenden Verbindungen wie Monoterpene, wie z. B. Thymol, Menthol oder Carvon, Diterpene, Triterpene wie z. B. Phytosterole (β-Sitosterol, Campesterol, Stigmasterol), Cardenolide, Tetraterpene wie z. B. Carotine.

Glucosinolate. Glucosinolate sind β-S-Glucoside von Thiohydroxamsäuren, wie z.B. Sinigrin, Sinalbin oder Glucobrassicin.

Sulfide. Zu dieser Gruppe gehören z.B. Alliin und Allicin.

Beispielhaft seien folgende Pflanzen genannt, die reich an einer der oben genannten Gruppe von sekundären Pflanzeninhaltsstoffen sind: Aesculus hippocastanum, Althaea, Allium cepa, Brassica nigra, Camellia sinensis, Carum carvi, Cimicifuga racemosa, Crataegus oxyacantha, Echinaceae purpurea, Ginkgo biloba, Glycine max, Hedera helix, Humulus lupulus, Hypericum perforatum, Linum usitatissimum, Mentha piperita, Myrtus communis, Opuntia ficus-indica, Panax ginseng, Silybum marianum, Trifolium pratense, Vaccinium myrtillus, Vitex agnus-castus und Vitis vinifera.

Die Kenntnis über die gesundheitsfördernden Wirkungen der sekundären Pflanzeninhaltsstoffe hat in den letzten Jahren stark zugenommen. In zahlreichen epidemiologischen oder auch in-vitro Studien zeigen sich folgende Wirkungen: antikanzerogen, antimikrobiell, antioxidativ, antithrombotisch, immunmodulierend, entzündungshemmend, Blutdruck-beeinflussend, Cholesterin-senkend, Blutglucosebeeinflussend und verdauungsfördernd (Watzl B., Leitzmann C. (1999) Bioaktive Substanzen in Lebensmitteln, Hippokrates Verlag).

Die Daten zur Bioverfügbarkeit der verschiedenen Pflanzeninhaltsstoffe sind noch lückenhaft. Für das Flavonoid Quercetin wurde bei isolierter Gabe eine Resorption von 24 % ermittelt, während die Resorption von in Zwiebeln vorliegendem Quercetin bei 52 % liegt (Hollmann P. C. H., de Vries J. H. M., van Leuwen S. D., Mengelers M. J. B., Katan M. B. (1995) Absorption of dietary quercetin glycosides and quercetin in healthy ileostomy volunteers. Am. J. Clin. Nutr. 62, 1276-1282; Hollmann P. C. H., Katan M. B.(1999) Dietary flavonoids: Intake, health effects and bioavailability. Food Chem. Toxicology 37, 937-942). 24 Stunden nach oraler Aufnahme von Flavanolen, wie Epigallocatechingallat, wurden im Tierversuch etwa ein Drittel der Dosis mit den Faeces ausgeschieden (Sugunuma M., Okabe S., Oniyama M., Tada Y., Ito H., Fijiki H. (1998) Wide distribution of 3H-epigallocatechin gallate, a cancer preventive tea polyphenol, in mouse tissue, Carcinogenesis 19, 1771-1776). Für Isoflavonoide wurde eine Bioverfügbarkeit von 13 - 35 % ermittelt (Xu X., Harris K. S., Wang H. J., Murphy P. A., Hendrich S.(1995) Bioavailability of soybean isoflavones depends upon gut microflora in women. J. Nutr. 125, 2307-2315). Insgesamt ist, soweit bekannt, die Bioverfügbarkeit der sekundären Pflanzenstoffe nicht sehr hoch.

Grundsätzlich erfolgt die Resorption durch die Lipiddoppelschicht der Schleimhaut des Magen-Darm-Kanals durch passive Diffusion, durch Carrier-vermittelte Diffusion, durch aktiven Transport und durch Pinozytose/Phagocytose/Persorption. Quantitativ gesehen steht die Diffusion durch die Lipidmatrix im Vordergrund, daher kommt der Lipidlöslichkeit der zu resorbierenden Substanz eine dominierende Rolle zu. Sehr polare Stoffe, wie Aminosäuren, Zucker und wasserlösliche Vitamine werden durch einen aktiven Transport resorbiert. Wichtig ist in diesem Zusammenhang das Transportprotein p-Glykoprotein in der Darmschleimhaut, das die Aufgabe hat unerwünschte resorbierte Fremd-Substanzen wieder in das Lumen des Gastrointestinaltraktes auszuschleusen. Ebenso steht einer Resorption eine mögliche Metabolisierung durch Cytochrom P450-Enzyme der Enterocyten im Wege.

Mit anderen Worten ist der Resorptionsapparat so konstruiert, daß primär Nahrungsbestandteile wie Eiweiß, Fett und Kohlenhydrate, daneben aber auch Vitamine und Mineralstoffe resorbiert werden. Da das Polaritätsspektrum der genannten Verbindungen von apolar bis polar reicht, müssen und werden Verbindungen durch verschiedene Mechanismen resorbiert, aber immer mit dem Ziel der Nahrungsaufnahme. Nicht der Nahrung dienende, aber strukturverwandte Verbindungen werden teilweise auch resorbiert, natürlich auch andere Fremdsubstanzen, die entsprechende physiko-chemische Eigenschaften aufweisen. D. h. sekundäre Pflanzenstoffe, die nicht als primäre Nährstoffe Verwendung finden, werden vom Körper mehr oder weniger schlecht resorbiert.

Da die Bioverfügbarkeit Voraussetzung einer physiologischen oder pharmakologischen Wirkung ist, ist eine Erhöhung der Bioverfügbarkeit der sekundären Pflanzeninhaltsstoffe wünschenswert und Aufgabe der vorliegenden Erfindung.

Diese erfindungsgemäße Aufgabe wird gelöst durch die Verwendung nach Anspruch 1 sowie durch das Verfahren nach Anspruch 9.

Bekannt ist, daß apolare Verbindungen, wie z. B. Carotin oder Lycopin bei einer fettreichen Mahlzeit besser resorbiert werden als bei einer fettarmen Mahlzeit. Überraschend wurde nun gefunden, daß auch polare Verbindungen, wie z. B. Flavone auch bei gleichzeitiger Gabe von Fett besser resorbiert werden. Insbesondere die Verwendung von omega-3-Fettsäuren erwies sich als vorteilhaft. Darüber hinaus wurde die unerwartete Beobachtung gemacht, dass empfindliche Pflanzeninhaltsstoffe im Gemisch mit den genannten Ölen stabilisiert werden, der Abbau also nicht oder zumindest mit verminderter Geschwindigkeit stattfindet.

Geeignete Öle mit einem Gehalt an omega-3-Fettsäuren sind z. B. Fischöl, Leinöl oder Perillasamenöl.

Bevorzugt sind Zusammensetzungen, die eine der nachfolgenden Kombinationen enthalten: Extrakt aus Opuntia ficus-indica und Perillasamenöl, Extrakt aus Vitis vinifera und Perillasamenöl, Extrakt aus Humulus lupulus und Leinöl, Extrakt aus Ginkgo biloba und Leinöl, Extrakt aus Hypericum perforatum und Fischöl, Extrakt aus Camellia sinensis und Fischöl, Extrakt aus Silybum marianum und Perillasamenöl, Extrakt aus Vitex agnus castus und Perillasamenöl, Extrakt aus Vaccinium myrtillus und Perillasamenöl, Extrakt aus Trifolium pratense und Perillasamenöl, Extrakt aus Myrtus communis und Perillasamenöl, Extrakt aus Mentha piperita und Perillasamenöl, Extrakt aus Linum usitatissimum und Perillasamenöl, sowie Extrakt aus Cimicifuga racemosa und Perillasamenöl.

Die Extrakte können nach an sich bekannten Herstellungsverfahren in variabler Zusammensetzung mit Lösungsmitteln wie z. B. Wasser, Methanol, Ethanol, 2-Propanol, Aceton, etc. und deren Gemischen, bei Temperaturen von Raumtemp. bis 100 °C unter gelinder bis heftiger Durchmischung oder durch Perkolation innerhalb von 10 Min. bis 24 Std. unter Normaldruck oder erhöhtem Druck erhalten werden. Zur Anreicherung von wirksamkeitsrelevanten Komponenten können weitere Konzentrierungsschritte durchgeführt werden wie z. B. flüssigflüssig-Verteilung mit z. B. 1-Butanol/Wasser oder Ethylacetat/Wasser, Adsorption-Desorption an Ionenaustauscher, LH20, HP20 und andere Harze oder chromatographische Abtrennungen über RP18, Kieselgel, etc.. Die Weiterverarbeitung zu Trockenextrakten erfolgt nach an sich bekannten Verfahren durch Abziehen des Lösungsmittels bei erhöhter Temperatur und/oder reduziertem Druck.

Zur Herstellung oral verabreichbarer Darreichungsformen wird ein Pflanzenextrakt mit einem omega-3-fettsäurenhaltigen Öl vermischt und ggf. unter Zusatz von Hilfstoffen wie z. B. Stabilisatoren, Füllmittel etc., in Kapseln abgefüllt.

### Beispiele:

Der Pflanzenextrakt wird mit dem Öl (beide gemäß nachstehender Tabelle) gemischt und die erhaltene fließfähige Suspension wird mit einem geeigneten an sich bekannten Verfahren in Kapseln abgefüllt.

### Beispiel 1

| | Bestandteile | mg/Kapselfüllung |
|---|---|---|
| 1 | Extrakt aus Rotwein (Vitis vinifera) | 100,0 |
| 2 | Perillasamenöl | 450,0 |

### Beispiel 2

| | Bestandteile | mg/Kapselfüllung |
|---|---|---|
| 1 | Extrakt aus Hopfenblüten (Humulus lupulus) | 100,0 |
| 2 | Leinöl | 450,0 |

### Beispiel 3

| | Bestandteile | mg/Kapselfüllung |
|---|---|---|
| 1 | Extrakt aus Ginkgo biloba | 100,0 |
| 2 | Leinöl | 450,0 |

### Beispiel 4

| | Bestandteile | mg/Kapselfüllung |
|---|---|---|
| 1 | Extrakt aus Opuntia ficus-indica | 100,0 |
| 2 | Perillasamenöl | 450,0 |

Die Bioverfügbarkeit der in den Pflanzenextrakten gemäß den Beispielen 1 bis 4 enthaltenen Flavonoide ist in den Zubereitungen der Beispiele 1 bis 4 erhöht gegenüber Kapseln, in denen nur der entsprechende Pflanzenextrakt, nicht jedoch das omega-3-fettsäurehaltige Öl enthalten ist.

### Beispiel 5

Männliche Ratten (Sprague Dawley) erhielten 300 mg/kg Trockenextrakt aus Blüten von Opuntia ficus-indica (Gesamtextrakt hergestellt durch Extraktion mit 60 Gew. % Ethanol bei 50°C bis 60°C und nachfolgender Filtration und Trocknung) oral per Schlundsonde in 0,2 % Agar suspendiert verabreicht. Nach 2, 4, 8, 24, 30 und 48 h wurden je 6 Tiere getötet und Plasma gewonnen. In der gleichen Weise erhielt eine andere Gruppe 200 mg/kg Perillasamenöl und sofort anschließend 300 mg/kg des oben genannten Opuntia-Extraktes.

Die Plasmaproben wurden nach enzymatischer Spaltung durch Glucuronidase mit tert.-Butylmethylether (TBME) extrahiert und das Flavonol Quercetin per HPLC bestimmt.

Probenvorbereitung: 800 µl Plasma, 30 µl Vitamin C (0.5 % in Wasser), 80 µl Essigsäure 0.5 M und 100 µl Glucuronidase aus Helix pomatia (2000 Units Glucuronidase; gelöst in Wasser) in 600 µl Aceton wurden 1 h bei 37 °C gehalten und anschließend mit 4 ml TBME extrahiert. Das organische Lösungsmittel wurde abgedampft und der Rückstand in HPLC-Eluens (40 % Methanol / 60 % Wasser /Phosphorsäure 85 % (pH 2)) aufgenommen.

### HPLC-Bedingungen:

| | |
|---|---|
| Säule: | RP18, Kromasil, 125 x 4 mm |
| Eluens: | A: Wasser / Phosphorsäure 85 % (pH 2) B: Methanol |
| Gradient: | 0 bis 1 min: 60 % A und 40 % B 1 bis 20 min: Anteil B wird von 40 auf 55 % erhöht |
| Detektion: | UV (370 nm) |
| Fluss: | 1 ml/min |

### Ergebnis

Die Plasmakonzentrationen an Quercetin sind in Tabelle 1 zusammengefasst.

| Zeit (h) | Quercetin (ng/ml) | |
|---|---|---|
| | Opuntia-Extrakt mit Perillasamenöl | Opuntia-Extrakt ohne Perillasamenöl |
| 2 | 251 | 159 |
| 4 | 317 | 239 |
| 8 | 116 | 94 |
| 24 | 66 | 33 |
| 30 | 47 | 33 |
| 48 | 44 | 27 |

Die Plasmaspiegel an Quercetin, einem der mit am weitesten verbreiteten Flavonoide, sind bei gleichzeitiger Gabe von Perillasamenöl höher. Die Fläche unter der Kurve (AUC ₍₀₋₄₈ₕ₎) beträgt 4290 ng/ml.h nach Gabe von Opuntia-Extrakt und Perillasamenöl und nur 2973 ng/ml.h nach Gabe von Opuntia-Extrakt ohne Perillasamenöl. Damit erhöht sich die Bioverfügbarkeit von Quercetin nach Gabe von Opuntia-Extrakt und Perillasamenöl um 44 % im Vergleich zur Gabe ohne Perillasamenöl.

## Patentansprüche

1. Verwendung von omega-3-fettsäurehaltigen Ölen zur Erhöhung der Bioverfügbarkeit von sekundären Pflanzeninhaltsstoffen aus Pflanzenextrakten.

2. Verwendung nach Anspruch 1, wobei das Öl ausgewählt ist aus Fischöl, Leinöl und Perillasamenöl.

3. Verwendung nach Anspruch 1 oder 2, wobei der Pflanzenextrakt sekundäre Pflanzeninhaltsstoffe enthält, ausgewählt aus der Gruppe umfassend Polyphenole, Isoprenoide, Glucosinolate und Sulfide.

4. Verwendung nach einem der Ansprüche 1 bis 3, wobei der Pflanzenextrakt Flavone als sekundäre Pflanzeninhaltsstoffe enthält.

5. Verwendung nach einem der Ansprüche 1 bis 4, wobei der Pflanzenextrakt ein Extrakt ist aus der Gruppe der folgenden Pflanzen: Aesculus hippocastanum, Althaea, Allium cepa, Brassica nigra, Camellia sinensis, Carum carvi, Cimicifuga racemosa, Crataegus oxyacantha, Echinaceae purpurea, Ginkgo biloba, Glycine max, Hedera helix, Humulus lupulus, Hypericum perforatum, Linum usitatissimum, Mentha piperita, Myrtus communis, Opuntia ficus-indica, Panax ginseng, Silybum marianum, Trifolium pratense, Vaccinium myrtillus, Vitex agnus-castus und Vitis vinifera.

6. Verwendung nach einem der Ansprüche 1 bis 5, wobei der Pflanzenextrakt und das omega-3-fettsäurehaltige Öl in einem diätetischen Nahrungsmittel oder Arzneimittel enthalten sind.

7. Verwendung nach einem der Ansprüche 1 bis 6 zur Erhöhung der Bioverfügbarkeit bei oraler Verabreichung.

8. Verwendung nach Anspruch 7 zur Erhöhung der Bioverfügbarkeit bei oraler Verabreichung in Form einer Kapsel.

9. Verfahren zur Erhöhung der Bioverfügbarkeit von sekundären Pflanzeninhaltsstoffen aus Pflanzenextrakten, **dadurch gekennzeichnet, dass** sie zusammen mit omega-3-fettsäurehaltigen Ölen verabreicht werden.

10. Verfahren nach Anspruch 9, wobei das Öl ausgewählt ist aus Fischöl, Leinöl und Perillasamenöl.

11. Verfahren nach Anspruch 9 oder 10, wobei der Pflanzenextrakt sekundäre Pflanzeninhaltsstoffe enthält, ausgewählt aus der Gruppe umfassend Polyphenole, Isoprenoide, Glucosinolate und Sulfide.

12. Verfahren nach einem der Ansprüche 9 bis 11, wobei der Pflanzenextrakt Flavone als sekundäre Pflanzeninhaltsstoffe enthält.

13. Verfahren nach einem der Ansprüche 9 bis 12, wobei der Pflanzenextrakt ein Extrakt ist aus der Gruppe der folgenden Pflanzen: Aesculus hippocastanum, Althaea, Allium cepa, Brassica nigra, Camellia sinensis, Carum carvi, Cimicifuga racemosa, Crataegus oxyacantha, Echinaceae purpurea, Ginkgo biloba, Glycine max, Hedera helix, Humulus lupulus, Hypericum perforatum, Linum usitatissimum, Mentha piperita, Myrtus communis, Opuntia ficus-indica, Panax ginseng, Silybum marianum, Trifolium pratense, Vaccinium myrtillus, Vitex agnus-castus und Vitis vinifera.

14. Verfahren nach einem der Ansprüche 9 bis 13, wobei der Pflanzenextrakt und das omega-3-fettsäurehaltige Öl in einem diätetischen Nahrungsmittel oder Arzneimittel enthalten sind.

15. Verfahren nach einem der Ansprüche 9 bis 14 zur Erhöhung der Bioverfügbarkeit bei oraler Verabreichung.

16. Verfahren nach einem der Ansprüche 9 bis 15 zur Erhöhung der Bioverfügbarkeit bei oraler Verabreichung in Form einer Kapsel.

## Claims

1. Use of omega-3 fatty acid-containing oils for increasing the bioavailability of secondary plant ingredients from plant extracts.

2. The use according to claim 1, said oil being selected from fish oil, linseed oil and perilla seed oil.

3. The use according to claim 1 or 2, said plant extract containing secondary plant ingredients selected from the group comprising polyphenols, isoprenoids, glucosinolates and sulfides.

4. The use according to any of the claims 1 to 3, said plant extract containing flavones as secondary plant ingredients.

5. The use according to any of the claims 1 to 4, said plant extract being an extract from the group of the following plants: *Aesculus hippocastanum, Althaea, Allium cepa, Brassica nigra, Camellia sinensis, Carum carvi, Cimicifuga racemosa, Crataegus oxyacantha, Echinaceae purpurea, Ginkgo biloba, Glycine max, Hedera helix, Humulus lupulus, Hypericum perforatum, Linum usitatissimum, Mentha piperita, Myrtus communis, Opuntia ficus-indica, Panax ginseng, Silybum marianum, Trifolium pratense, Vaccinium myrtillus, Vitex agnus-castus* and *Vitis vinifera.*

6. The use according to any of the claims 1 to 5, said plant extract and the omega 3 fatty acid-containing oil being contained in a dietetic food or pharmaceutical preparation.

7. The use according to any of the claims 1 to 6 for increasing the bioavailability upon oral administration.

8. The use according to claim 7 for increasing the bioavailability upon oral administration in the form of a capsule.

9. A method for increasing the bioavailability of secondary plant ingredients from plant extracts, **characterised in that** they are administered together with omega-3 fatty acid-containing oils.

10. The method according to claim 9, said oil being selected from fish oil, linseed oil and perilla seed oil.

11. The method according to claim 9 or 10, said plant extract containing secondary plant ingredients selected from the group comprising polyphenols, isoprenoids, glucosinolates and sulfides.

12. The method according to any of the claims 9 to 11, said plant extract containing flavones as secondary plant ingredients.

13. The method according to any of the claims 9 to 12, said plant extract being an extract from the group of the following plants: *Aesculus hippocastanum, Althaea, Allium cepa, Brassica nigra, Camellia sinensis, Carum carvi, Cimicifuga racemosa, Crataegus oxyacantha, Echinaceae purpurea, Ginkgo biloba, Glycine max, Hedera helix, Humulus lupulus, Hypericum perforatum, Linum usitatissimum, Mentha piperita, Myrtus communis, Opuntia ficus-indica, Panax ginseng, Silybum marianum, Trifolium pratense, Vaccinium myrtillus, Vitex agnus-castus* and *Vitis vinifera.*

14. The method according to any of the claims 9 to 13, said plant extract and the omega 3 fatty acid-containing oil being contained in a dietetic food or pharmaceutical preparation.

15. The method according to any of the claims 9 to 14 for increasing the bioavailability upon oral administration.

16. The method according to any of the claims 9 to 15 for increasing the bioavailability upon oral administration in the form of a capsule.

## Revendications

1. Utilisation d'huiles contenant des acides gras oméga-3 pour élever la biodisponibilité de substances végétales secondaires d'extraits de plantes.

2. Utilisation selon la revendication 1, pour laquelle l'huile est choisie parmi l'huile de poissons, l'huile de lin et l'huile de graines de périlla.

3. Utilisation selon la revendication 1 ou 2, pour laquelle l'extrait de plante contient des substances végétales secondaires choisies dans le groupe comprenant les polyphénols, les isoprénoïdes, les glucosinolates et les sulfides.

4. Utilisation selon l'une des revendications 1 à 3, pour laquelle l'extrait de plante contient comme substances végétales secondaires, des flavones.

5. Utilisation selon l'une des revendications 1 à 4, pour laquelle l'extrait de plante est un extrait tiré du groupe des plantes suivantes : aesculus hippocastanum, althaea, allium cepa, brassica nigra, camellia sinensis, carum carvi, cimicifuga racemosa, crataegus oxyacantha, echinaceae purpurea, ginkgo biloba, glycine max, hedera helix, humulus lupulus, hypericum perforatum, linum usitatissimum, mentha piperita, myrtus communis, opuntia ficus-indica, panax ginseng, silybum marianum, trifolium pratense, vaccinium myrtillus, vitex agnus-castus et vitis vinifera.

6. Utilisation selon l'une des revendications 1 à 5, pour laquelle l'extrait de plante et l'huile contenant des acides gras oméga-3 sont contenus dans un produit alimentaire diététique ou un médicament.

7. Utilisation selon l'une des revendications 1 à 6 pour élever la biodisponibilité dans le cas d'une administration orale.

8. Utilisation selon la revendication 7 pour élever la biodisponibilité dans le cas d'une administration orale sous forme d'une capsule.

9. Procédé pour élever la biodisponibilité de substances végétales secondaires d'extraits de plante, **caractérisé en ce que** celles-ci sont administrées conjointement avec des huiles contenant des acides gras oméga-3.

10. Procédé selon la revendication 9, suivant lequel l'huile est choisie parmi l'huile de poissons, l'huile de lin et l'huile de graines de périlla.

11. Procédé selon la revendication 9 ou 10, suivant lequel l'extrait de plante contient des substances végétales secondaires choisies dans le groupe comprenant les polyphénols, les isoprénoïdes, les glucosinolates et les sulfides.

12. Procédé selon l'une des revendications 9 à 11, suivant lequel l'extrait de plante contient comme substances végétales secondaires, des flavones.

13. Procédé selon l'une des revendications 9 à 12, suivant lequel l'extrait de plante est un extrait tiré du groupe des plantes suivantes : aesculus hippocastanum, althaea, allium cepa, brassica nigra, camellia sinensis, carum carvi, cimicifuga racemosa, crataegus oxyacantha, echinaceae purpurea, ginkgo biloba, glycine max, hedera helix, humulus lupulus, hypericum perforatum, linum usitatissimum, mentha piperita, myrtus communis, opuntia ficus-indica, panax ginseng, silybum marianum, trifolium pratense, vaccinium myrtillus, vitex agnus-castus et vitis vinifera.

14. Procédé selon l'une des revendications 9 à 13, suivant lequel l'extrait de plante et l'huile contenant des acides gras oméga-3 sont contenus dans un produit alimentaire diététique ou un médicament.

15. Procédé selon l'une des revendications 9 à 14 pour élever la biodisponibilité dans le cas d'une administration orale.

16. Procédé selon l'une des revendications 9 à 15 pour élever la biodisponibilité dans le cas d'une administration orale sous forme d'une capsule.
